# EUROPÄISCHE PATENTSCHRIFT

(11) **EP 2 963 109 B1**
(45) Veröffentlichungstag und Bekanntmachung des Hinweises auf die Patenterteilung: **06.09.2017**
(21) Anmeldenummer: 14175312.9
(22) Anmeldetag: 01.07.2014
(51) Int. Cl.: C12N 9/02, C12N 9/90, C12P 7/26

(54) **Verfahren zur biotechnologischen Herstellung von Flavanonglykosid-Dihydrochalkonen**
Process for the biotechnological production of flavanonglycoside dihydrochalcones
Procedée pour la preparation biotechnologique du flavanon glycoside dihydrochalcones

(43) Veröffentlichungstag der Anmeldung: 06.01.2016
(73) Patentinhaber: Symrise AG, 37603 Holzminden (DE)
(72) Erfinder: Thomsen, Maren, 17489 Greifswald (DE); Bornscheuer, Uwe, 17489 Greifswald (DE); Hinrichs, Winfried, 17489 Greifswald (DE); Gross, Egon, 37603 Holzminden (DE); Ley, Jakob, 37603 Holzminden (DE); Geissler, Torsten, 37574 Einbeck (DE)
(74) Vertreter: Fabry, Bernd

(56) Entgegenhaltungen:
- EP-A1- 2 529 633
- EP-A1- 2 692 729
- US-A1- 2013 136 839
- MECHTHILD GALL ET AL: "Enzymatische Umsetzung von Flavonoiden mit einer bakteriellen Chalconisomerase und einer Enoatreduktase", ANGEWANDTE CHEMIE (INTERNATIONAL ED. IN ENGLISH), Bd. 126, Nr. 5, 27. Januar 2014 (2014-01-27), Seiten 1463-1466, XP055160309, ISSN: 0044-8249, DOI: 10.1002/ange.201306952

## Beschreibung

### GEBIET DER ERFINDUNG

Die Erfindung befindet sich auf dem Gebiet der Biotechnologie und betrifft ein Verfahren bei dem man Flavanonglykoside ohne chemische Zwischenschritte in die entsprechenden Dihydrochalkone umwandelt, einen entsprechenden Mikroorganismus, einen Vektor und eine Wirtszelle.

### STAND DER TECHNIK

Im Rahmen eines Forschungsprogramms des US Departments of Agriculture zur Verminderung des Bittergeschmacks in Zitrussäften wurden Mitte der 60er Jahres des vergangenen Jahrhunderts eine Reihe von Flavanonglykosiden wie beispielsweise Naringin, Hesperidin und Narirutin als die entscheidenden Bitterstoffe erkannt. Im Weiteren wurde gefunden, dass die Hydrierung der Flavanonglykoside zu Dihydrochalkonen zu Stoffen führt, die an der Nachweisgrenze des Süßungseffektes bis zu 1800mal süßer als Zucker schmecken; tauscht man Zucker gegen diese Dihydrochalkone im Gewichtsverhältnis 1:1 aus, erweisen sich die Dihydrochalkone immer noch um mindestens den Faktor 300 effizienter. Naringindihydrochalkon, Hesperidindihydrochalkon und Neohesperidindihydrochalkon zählen heute nicht nur zu den stärksten Süßstoffen, sondern sind auch in Besonderem dazu geeignet, den Bittergeschmack von Zitrusstoffen zu maskieren.

Bestimmte Dihydrochalkonglykoside lassen sich durch Extraktion aus Beeren, speziell aus *Malus spp.* erhalten. Das Verfahren ist jedoch aufwendig, kostspielig und zudem saisonabhängig, so dass es technisch keine Bedeutung hat.

Ausgehend von den Flavanonglykosiden erfolgt die Herstellung der Dihydrochalkone heute durch katalytische Reduktion unter stark basischen Bedingungen oder durch eine Friedel-Crafts-artige Acylierung von Phenolen mit Dihydrozimtsäuren. Auch wenn die Synthese in befriedigenden Ausbeuten erfolgt und industriell etabliert ist, weist sie jedoch einen entscheidenden Nachteil auf: es handelt sich um einen chemischen Herstellungsprozess, was bedeutet, dass das Endprodukt aus regulatorischen Gründen nicht als natürlich deklariert werden kann.

In diesem Zusammenhang soll die EP 2692729 A1 (SYMRISE) erwähnt werden, aus der ein Verfahren zur Herstellung eines Dihydrochalkons, unter Verwendung eines transgenen Mikroorganismus bekannt ist, das folgende Schritte umfasst:
(i) Bereitstellen eines transgenen Mikroorganismus, enthaltend einen Nukleinsäure-Abschnitt (a), umfassend oder bestehend aus einem für eine bakterielle Chalconisomerase kodierenden Gen, und/oder einen Nukleinsäure-Abschnitt (a'), umfassend oder bestehend aus einem für eine pflanzliche Chalconisomerase kodierenden Gen, sowie einen Nukleinsäure-Abschnitt (b), umfassend oder bestehend aus einem für eine bakterielle Enoat-Reduktase kodierenden Gen,
(ii) Hinzufügen von einem oder mehreren Flavanonen, und/oder von einer oder mehreren Vorstufen oder einem oder mehreren Derivaten davon, zu dem transgenen Mikroorganismus und Kultivieren des transgenen Mikroorganismus unter Bedingungen, die die Umsetzung des bzw. der Flavanone und/oder der Vorstufe(n) oder des Derivats bzw. der Derivate davon, zu einem Dihydrochalkon ermöglichen,
(iii) optional: Isolieren sowie gegebenenfalls Aufreinigung des Dihydrochalkons, insbesondere von Phloretin.

Gutachtlich zitiert wird der nachveröffentlichte Aufsatz von GALL et al mit dem Titel "Enzymatische Umsetzung von Flavonoiden mit einer bakteriellen Chalconisomerase und einer Enoatreduktase" in Angewandten Chemie, 126(5), S. 1463-1466 (2014**).** Beschrieben wird darin die Identifizierung und die rekombinante Expression der Chalkonisomerase und einer Enoat-Reduktase aus dem anaeroben Bakterium *Eubacterium ramulus.* Der die beiden Enzyme exprimierende *E.-coli* Stamm kann zur Durchführung der Umwandlung verschiedener Flavanone zu ihren jeweiligen Dihydrochalconen eingesetzt werden. Die Schrift offenbart damit lediglich ein Verfahren zur Herstellung Dihydrochalconen aus der entsprechenden Flavanone. Die Herstellung von *Flavanonglykosid*-Dihydrochalkonen wird in dieser Schrift nicht offenbart.

Die Aufgabe der vorliegenden Erfindung hat somit darin bestanden, ein Verfahren bereitzustellen, mit dessen Hilfe Flavanonglykoside rasch und in hohen Ausbeuten auf biotechnologischem Verfahren in die entsprechenden Dihydrochalkone überführt werden können, wobei chemische Zwischenschritte zwingend unterbleiben sollten.

### BESCHREIBUNG DER ERFINDUNG

Ein erster Gegenstand der Erfindung betrifft ein Verfahren zur Herstellung von Flavanonglykosid-Dihydrochalkonen, umfassend die Schritte:
(a) Bereitstellen eines transgenen Mikroorganismus, enthaltend
   (i) einen ersten Nukleinsäure-Abschnitt (A) enthaltend ein für eine bakterielle Chalkonisomerase kodierendes Gen, sowie
   (ii) einen zweiten Nukleinsäure-Abschnitt (B), enthaltend ein für eine bakterielle Enoat-Reduktase kodierendes Gen,
(b) Hinzufügen eines oder mehrere Flavanonglykoside zu dem transgenen Mikroorganismus,
(c) Kultivierung des transgenen Mikroorganismus unter Bedingungen, die die gleichzeitig Isomerisierung und Reduktion des Flavanonglykosids zum Flavanonglykosid-Dihydrochalkon ermöglichen, sowie gegebenenfalls
(d) Isolierung und Aufreinigung des Endproduktes.
wobei der Nukleinsäure-Abschnitt (A)
(1) eine Nukleinsäuresequenz gemäß SEQ ID NO:1 darstellt, bei der der Nukleinsäure-Abschnitt (A') gemäß SEQ ID NO:**3** herausgeschnitten worden ist, oder
(2) für eine Aminosäuresequenz gemäß SEQ ID NO:**2** kodiert, bei der der Aminosäuresäure-Abschnitt (A') gemäß SEQ ID NO:**4** herausgeschnitten worden ist.

Überraschenderweise wurde gefunden, dass durch Einbau von zwei unterschiedlichen Nukleinsäure-Abschnitten, die einmal ein für eine bakterielle Chalkonisomerase und zum anderen ein für eine bakterielle Enoat-Reduktase kodierendes Gen enthalten in einen geeigneten Mikroorganismus, vorzugsweise einen fakultativ anaeroben Mikroorganismus ein System zur Verfügung steht, welches bei Zugabe von Flavanonglykosiden und Kultivierung gleichzeitig die Isomerisierung des Flavanonglykosids zum Chalkon und die Reduktion des Chalkons zum Dihydrochalkons in kurzen Zeiten und ausgezeichneten Ausbeuten möglich macht, wie dies am Beispiel der Umsetzung des Flavanonglykosids Naringin zu Naringindihydrochalkon (= Naringin dihydrochalcone) exemplarisch dargestellt wird:

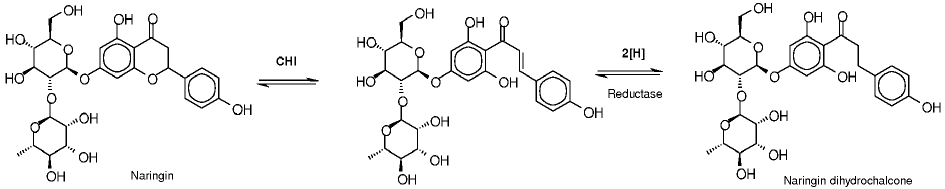

Da das gesamte Verfahren nur durch enzymatische/fermentative Prozesse auskommt, dürfen die so erhaltenen Flavanonglykosid-Dihydrochalkone als natürlich bezeichnet werden.

### CHALKONISOMERASEN

Eine "Chalkonisomerase" (CHI) im Sinne der vorliegenden Erfindung ist ein Enzym, das die Reaktion eines Flavanons zum Chalkon katalysiert. Insbesondere katalysiert die CHI die Reaktion von Naringin zu Naringinchalkon.

In einer konkreten Ausführungsform der Erfindung ist das vorliegende biotechnologische Verfahren dadurch gekennzeichnet, dass eine bakterielle Chalkonisomerase in Kombination mit einer ebenfalls bakteriellen Enoat-Reduktase, vorzugsweise aus dem gleichen Bakterium, in einen transgenen Mikroorganismus eingebracht wird, der daraufhin in der Lage ist, die gewünschten Flavanonglykosid-Dihydrochalkone, insbesondere Naringin Dihydrochalkon zu bilden.

Zu diesem Zweck kommt als erstes und insbesondere eine bakterielle Chalkonisomerase in Betracht, die aus einem Mikroorganismus aus dem Phylum der Firmicutes stammt, vorzugsweise der Klasse Clostridia, insbesondere der Ordnung Clostridiales wobei hierunter der anaerobe Organismus *Eubacterium ramulus* besonders bevorzugt ist.

Für die Zwecke der vorliegenden Erfindung besonders bevorzugt ist die Verwendung einer Chalkonisomerase, die eine, mehrere oder sämtliche der folgenden Eigenschaften aufweist:

| **K_{M} µmol/l]** | **Vₘₐₓ [U/mg]** | **k_{cat} [s⁻¹]** | **k_{cat}/ K_{M} [l*mol⁻¹*s⁻¹]** |
|---|---|---|---|
| 36,83 | 107,3 | 416,7 | 1,13 * 107 |

Aus dem Stand der Technik ist bekannt, dass der anaerobe Mikroorganismus *Eubacterium ramulus* in der Lage ist, Naringenin abzubauen, wobei intermediär Phloretin gebildet wird. Das intermediär gebildete Phloretin wird in *Eubacterium ramulus* unmittelbar weiter verstoffwechselt (vgl. Schneider et al, Anaerobic degradation of flavonoids by Eubacterium ramulus, Arch Microbiol (2000) 173 : 71-75**),** wie in dem folgenden Reaktionsschema dargestellt ist (siehe insbesondere die durch die Phloretinhydrolase (PhH) vermittelte Reaktion):

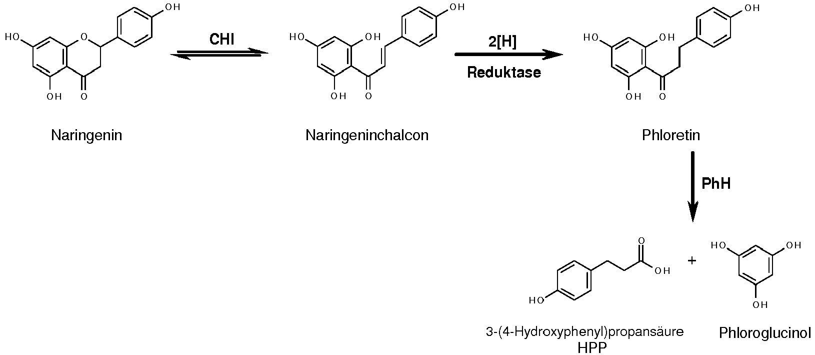

Im Stand der Technik sind zwar unterschiedliche Einsatzmöglichkeiten von Enzymsystemen und Methoden der mikrobiellen Biotransformation beschrieben; bekannt ist beispielsweise die Möglichkeit einer einfachen Reduktion von Doppelbindungen mittels Hefen (z.B. Saccharomyces). Enzymatische Etherspaltungen wurden bisher jedoch kaum untersucht.

Im Zusammenhang mit der vorliegenden Erfindung sei grundsätzlich auf folgende Veröffentlichungen verwiesen: Schoefer et al, Anaerobic Degradation of Flavonoids by Clostridium orbiscindens, Appl. Environ. Microbiol., Oct. 2003, p. 5849-5854**;** und Her/es et al, "First bacterial chalcone isomerase isolated from Eubacterium ramulus", Arch Microbiol (2004) 181: 428-434**.** Erkenntnisse im Zusammenhang mit dem Abbau von Lignin sind beispielsweise von Masai et al. beschrieben worden (Masai et al., 1993; Otsuka et al., 2003; s. auch JP 2002034557**).**

In WO 2006 010117 A1 (KOFFAS) und WO 2005 084305 A1 (SCHMIDT-DANERT) wird zudem die Anwendung heterologer Expression zur Bildung von Flavonoiden beschrieben. Darin werden (ausschließlich) pflanzliche Gene beschrieben, die für eine heterologe Expression verschiedener Substanzen (ausgehend von L-Phenylalanin, Tyrosin und Zimtsäure) offenbart.

Im Zusammenhang mit der vorliegenden Erfindung wurde jedoch festgestellt, dass Chalkonisomerasen im Allgemeinen und die CHI ex *E. ramulus* nicht immer befriedigende Produktausbeuten zeigen, wenn statt der Aglykone die entsprechenden Glykoside eingesetzt werden. Ein besonderer Gesichtspunkt der vorliegenden Erfindung hat somit darin bestanden, gezielte Veränderungen an dem Enzym vorzunehmen, so dass nun in praktisch gleicher Weise Aglykone oder die entsprechende Glykoside eingesetzt und die entsprechenden Dihydrochalkone dann auch in kurzer Zeit und in zufriedenstellenden Ausbeuten exprimiert werden.

Wie oben beschrieben betrifft die Erfindung ein Verfahren, welches sich dadurch auszeichnet, dass der in den transgenen Mikroorganismus eingebrachte Nukleinsäure-Abschnitt (A) eine Nukleotidsequenz gemäß SEQ ID NO:**1** enthält, bei der der Nukleinsäure-Abschnitt (A') gemäß SEQ ID NO:**3** herausgeschnitten worden ist.

Grundsätzlich geeignet sind auch solche Nukleinsäureabschnitte, die mit den erfindungsgemäßen Nukleinsäureabschnitten (A-A') zu mindestens 50 %, vorzugsweise zu mindestens 60 und insbesondere zu mindestens 80 % übereinstimmen, auch wenn sich mit diesen Sequenzen nicht die gleichen vorteilhaften Expressionszeiten und Expressionsausbeuten erzielen lassen.

Mutatis mutandis ist ebenfalls Gegenstand der Erfindung ein Verfahren, bei dem die bakterielle Chalkonisomerase eine Nukleotidsequenz gemäß SEQ ID NO:**1** enthält, bei der der Nukleinsäure-Abschnitt (A') gemäß SEQ ID NO:**3** herausgeschnitten worden ist.

Die vorliegende Erfindung betrifft weiterhin ein Verfahren, welches sich dadurch auszeichnet, dass der in den transgenen Mikroorganismus exprimierte Aminosäure-Abschnitt (A) eine Aminosäuresequenz gemäß SEQ ID NO:**2** enthält, bei der der Aminosäure-Abschnitt (A') gemäß SEQ ID NO:**4** herausgeschnitten worden ist.

Grundsätzlich geeignet sind auch solche Aminosäureabschnitte, die mit den erfindungsgemäßen Aminosäureabschnitten (A-A') zu mindestens 50 %, vorzugsweise zu mindestens 60 % und insbesondere zu mindestens 80 % übereinstimmen, auch wenn sich mit diesen Sequenzen nicht die gleichen vorteilhaften Expressionszeiten und Expressionsausbeuten erzielen lassen.

Mutatis mutandis ist ebenfalls Gegenstand der Erfindung ein Verfahren, bei dem die bakterielle Chalkonisomerase eine Aminosäuresequenz gemäß SEQ ID NO:**2** enthält, bei der der Aminosäure-Abschnitt (A') gemäß SEQ ID NO:**4** herausgeschnitten worden ist.

Im Rahmen der vorliegenden Erfindung ist die "Aminosäuresequenz-Identität" bevorzugt anhand des Waterman-Smith-Algorithmus mit einer Lückeneröffnungs-Strafe ("gap o-pen penalty") von 10, einer Lückenvergrößerungsstrafe ("gap extension penalty") von 0,5 und der BLOSUM62-Matrix zu bestimmen (bezüglich des Waterman-Smith-Algorithmus siehe beispielsweise Smith, T.F. and Waterman, M.S., "Identification of common molecular subsequences", Journal of Molecular biology (1981), 147:195-197; online implementiert über die entsprechende Werkzeugseite des EMBL). Die vorgelegten Nukleotid-Sequenzen wurden mit Hilfe der Software BISSAP des Europäischen Patentamtes gemäß WIPO Standard 25 erzeugt

Das Entfernen des genannten Abschnitts aus der Gesamtsequenz kann beispielsweise mit der FastCloning Methode durchgeführt werden. Zu diesem Zweck wird eine PCR mit folgenden Primern durchgeführt:
Forward Primer: 5'-GATCCCGGCAGCAGCAGAAGGAAATCC-3'
Reverse Primer: 5'- GGATTTCCTTCTGCTGCTGCCGGGATC-3'

Anschließend wird das Ausgangs-Plasmid mit Dpnl. Nach erfolgreicher Klonierung kann das so erhaltene Plasmid pET28_CHI_ΔLid zusammen mit dem zweiten Plasmid pET22_ERED (wie im Folgenden näher erläutert) in einen Mikroorganismus, hier vorzugsweise *E. coli* BL21 in an sich bekannter Weise transformiert werden.

### ENOAT REDUKTASEN

Eine "Enoat-Reduktase" (ERED) im Sinne der vorliegenden Erfindung ist ein Enzym, das die Dehydrierung bestimmter Verbindungen katalysiert, insbesondere die Reaktion von Naringeninglykosidchalkon zu Naringeninglykosiddihydrochalkon.

Als zweite und ebenfalls bevorzugte Ausführungsform kommt eine bakterielle Enoat-Reduktase aus einem Mikroorganismus aus dem Phylum der Firmicutes in Betracht, vorzugsweise der Klasse Clostridia, insbesondere der Ordnung Clostridiales, wobei hierunter der anaerobe Organismus *Eubacterium ramulus* besonders bevorzugt ist. Insgesamt bevorzugt ist die Doppeltransformation sowohl der Chalkonisomerase als auch der Enoat-Reduktase ex *Eubacterium ramulus* in einen geeigneten Mikroorganismus, vorzugsweise *E. coli.*

Für die Zwecke der vorliegenden Erfindung bevorzugt ist die Verwendung einer Enoat-Reduktase, die eine Proteingröße von 74,455 kDa aufweist und/oder sowohl in der löslichen als auch in der unlöslichen Proteinfraktion nach bis zu 20h unter anoxischen Bedingungen bei verschiedenen Temperaturen exprimiert.

Eine weitere besondere Ausführungsform der Erfindung betrifft daher ein Verfahren welches sich dadurch auszeichnet, dass der in den transgenen Mikroorganismus eingebrachte Nukleinsäure-Abschnitt (B) eine Nukleotidsequenz gemäß SEQ ID NO:**5** oder SEQ ID NO:**7** darstellt.

Mutatis-mutandis ist ebenfalls Gegenstand der Erfindung ein Verfahren, bei dem die bakterielle Enoat-Reduktase eine Nukleotidsequenz gemäß SEQ ID NO:**5** oder SEQ ID NO:**7** enthält.

Eine weitere besondere Ausführungsform der Erfindung betrifft daher ein Verfahren welches sich dadurch auszeichnet, dass der in den transgenen Mikroorganismus exprimierte Aminosäure-Abschnitt (B) eine Aminosäuresequenz gemäß SEQ ID NO:**6** darstellt.

Grundsätzlich geeignet sind auch solche Aminosäureabschnitte, die mit den erfindungsgemäßen Aminosäureabschnitten (B) zu mindestens 50 %, vorzugsweise zu mindestens 60 % und insbesondere zu mindestens 80 % übereinstimmen, auch wenn sich mit diesen Sequenzen nicht die gleichen vorteilhaften Expressionszeiten und Expressionsausbeuten erzielen lassen.

Mutatis-mutandis ist ebenfalls Gegenstand der Erfindung ein Verfahren, bei dem die bakterielle Enoat-Reduktase eine Aminosäuresequenz gemäß SEQ ID NO:**6** enthält.

### FLAVANONGLYKOSIDE

Im Sinne der vorliegenden Erfindung kommen als Flavanonglykoside, die zu den entsprechenden Flavanonglykosid-Dihydrochalkonen umgesetzt werden sollen, die folgenden Stoffe in Betracht:

Naringin, Narirutin, Prunin (Naringin-7-O-glucosid), Hesperidin, Neohesperidin, Hesperetin-7-O-glucosid, Eriodictyolglycoside wie Eriocitrin, Neoeriocitrin, Eriodictyol-7-O-glucosid, Homoeriodictyolglycoside wie Homoeriodictyol-7-O-glucosid, Sterubinglycoside, Sakuranetinglycoside, Isosakuranetinglycoside, 4',7-Dihydroxy-flavanonglykoside, 4',7-Dihydroxy-3'-methoxy-flavanonglycoside, 3',7-Dihydroxy-4'-methoxy-flavanonglycoside, 3',4',7-Trihydroxy-flavanonglycoside, wobei die Flavanone bezüglich der 2-Position des Flavanongerüsts als (S)-, als (R)-Enantiomer, als Racemat oder als beliebige Mischung der beiden Enantiomere vorliegen können. Im Folgenden sind einige der bevorzugt einzusetzenden Flavanone exemplarisch abgebildet:

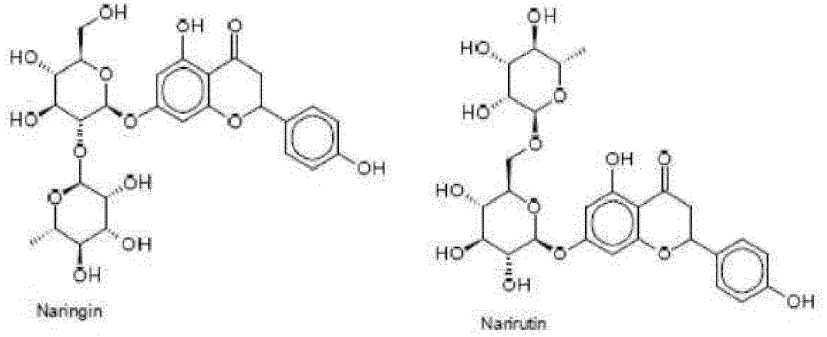

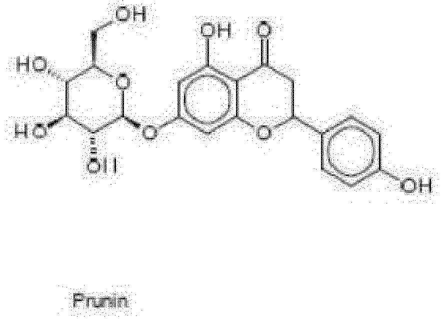

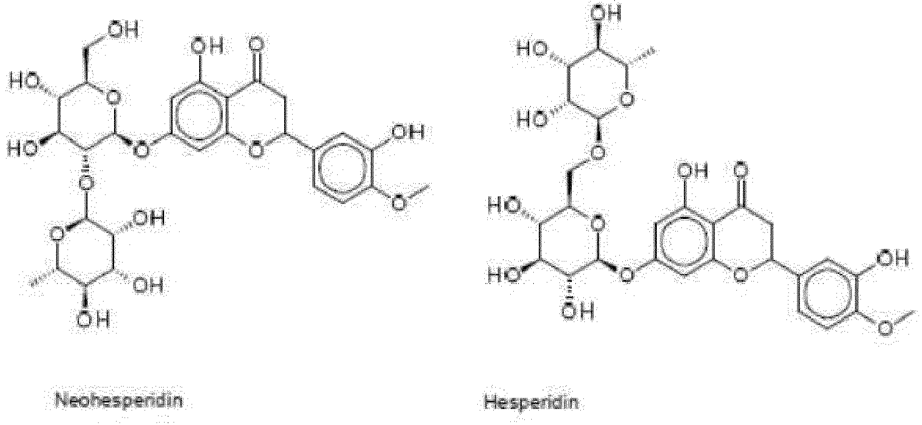

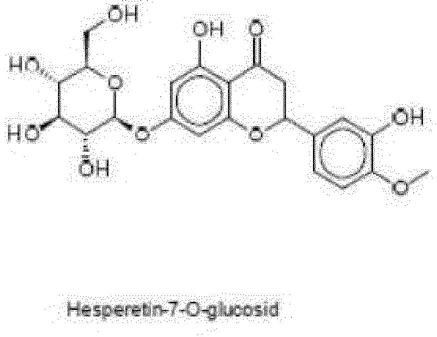

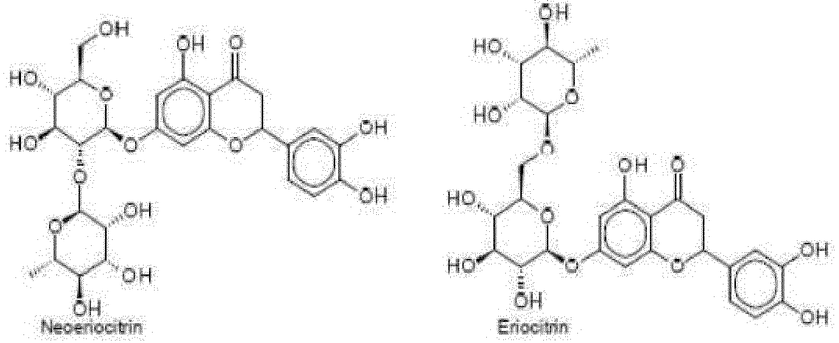

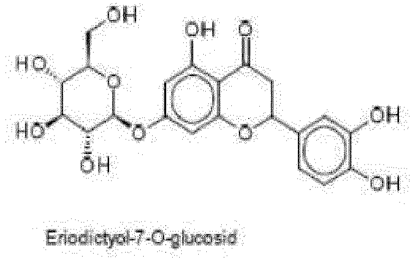

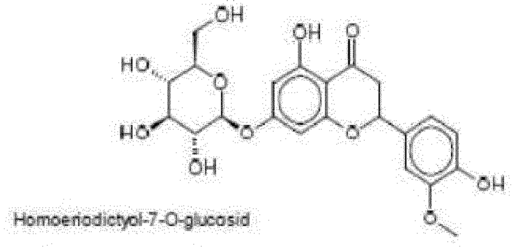

Diese lassen sich gemäß dem Verfahren der Erfindung in die entsprechenden Dihydrochalkone umwandeln, die nachstehend wiedergegeben sind:

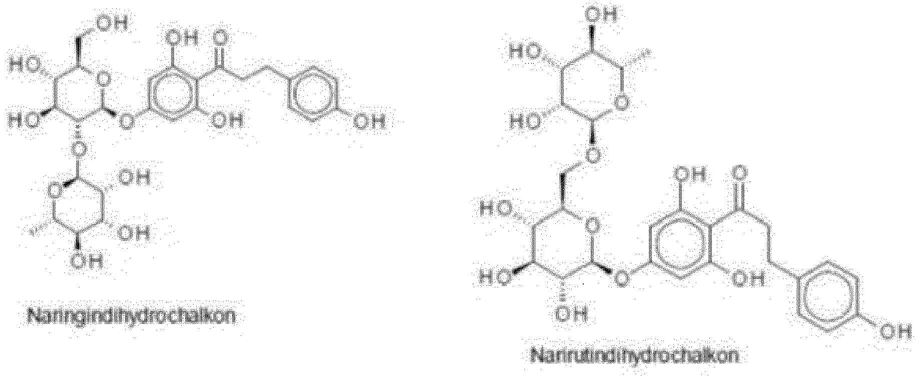

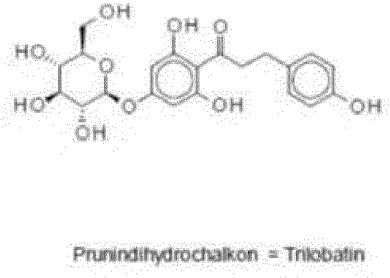

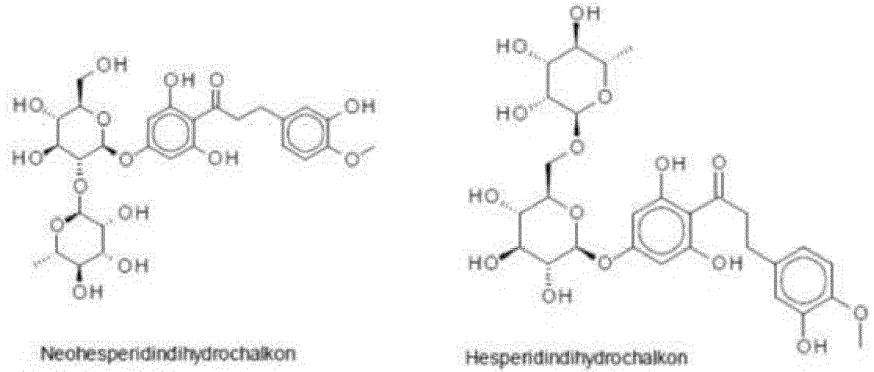

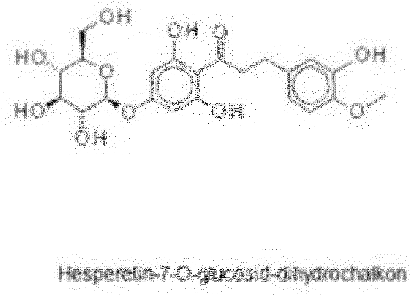

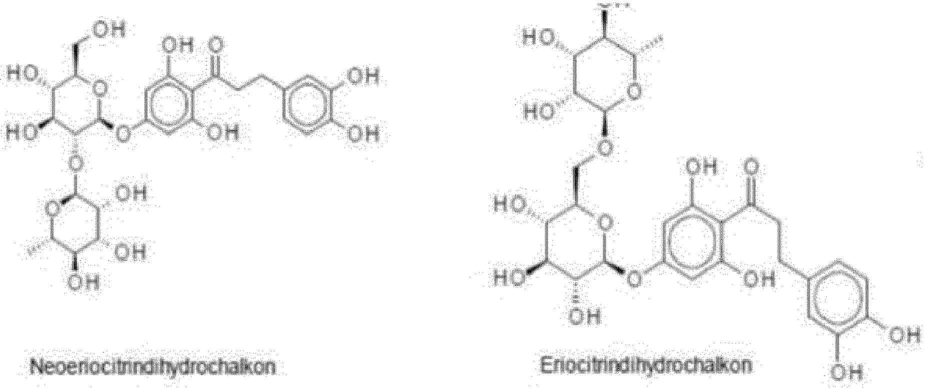

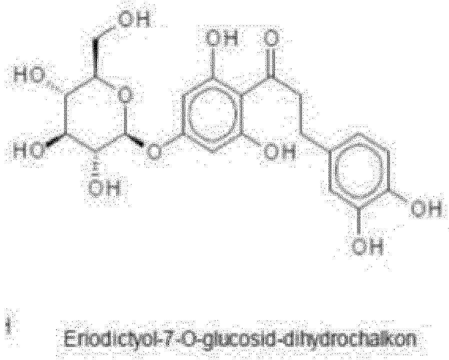

Besonders bevorzugt - weil kommerziell von erheblicher Bedeutung und in besonders kurzen Zeiten und hohen Ausbeuten erhältlich - ist die Umsetzung der folgenden Flavanonglykoside zu den entsprechenden Dihydrochalkonen:

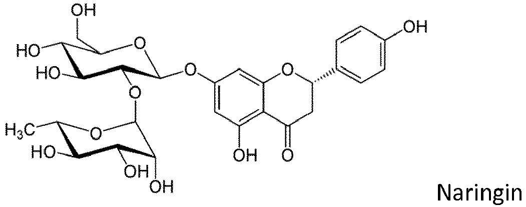

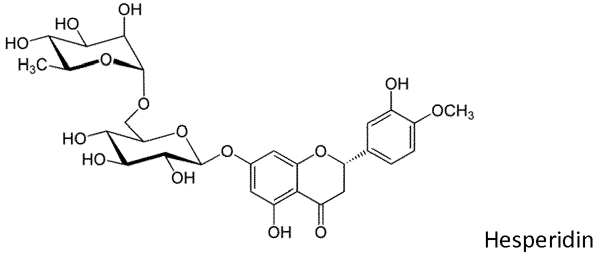

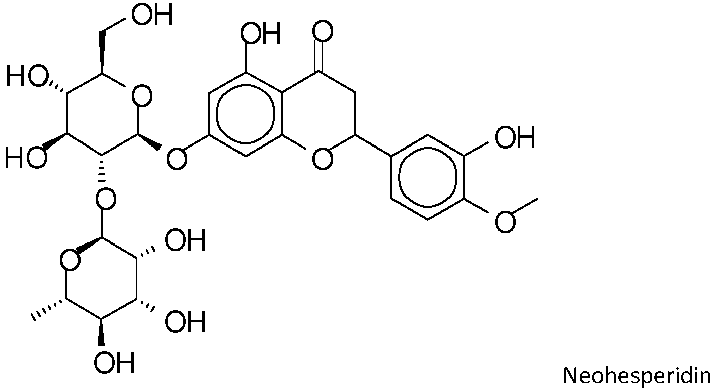

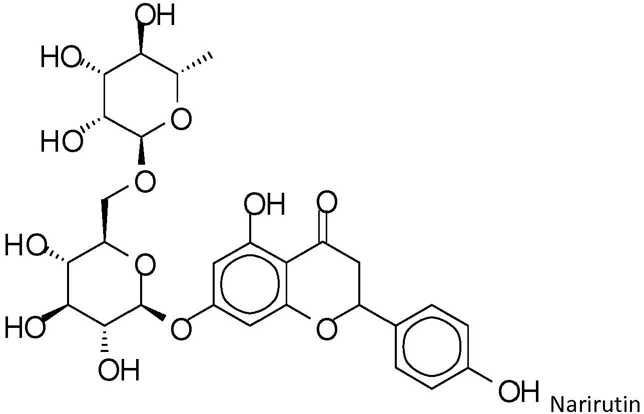

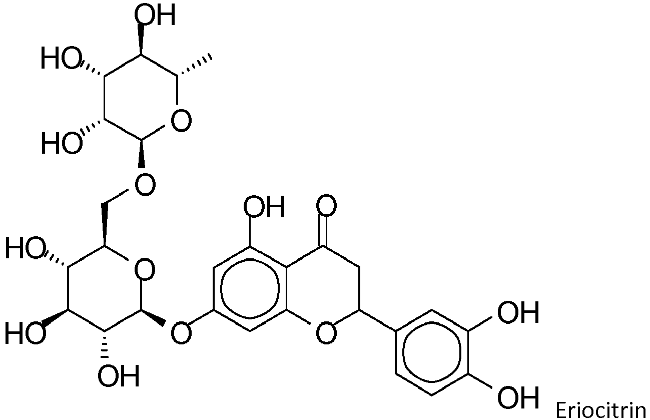

### KULTIVIERUNG; EXPRESSION UND ISOLIERUNG

Wie oben beschrieben wird bzw. werden in Schritt (ii) eines erfindungsgemäßen Verfahrens ein oder mehrere Flavanonglykoside zu dem transgenen Mikroorganismus hinzugefügt, wobei der transgene Mikroorganismus unter Bedingungen kultiviert wird, die die Umsetzung des bzw. der Flavanonglykoside ermöglichen.

Gemäß einer bevorzugten Durchführung eines erfindungsgemäßen Verfahrens werden die transgenen Mikroorgansimen zunächst, d.h. vor Schritt (ii), unter aeroben Bedingungen, vorzugsweise bis zum Erreichen einer maximalen Biomassenkonzentration, kultiviert. Dabei sollte die OD₆₀₀ bevorzugt wenigstens im Bereich von 1 bis 15 oder darüber liegen, vorzugsweise im Bereich von 5 bis 300, insbesondere im Bereich von 10 bis 275, bevorzugt im Bereich von 15 bis 250. Anschließend werden die Mikroorganismen in Schritt (ii) bevorzugt unter anaeroben Bedingungen kultiviert, wobei die Expression der gewünschten Aminosäuresequenzen bzw. der gewünschten Enzyme auf Basis der eingeschleusten Nukleinsäure-Abschnitte bzw. der eingeschleusten Transgene erfolgt, beispielsweise angeregt mittels Induktion durch IPTG und/oder Lactose (bei Verwendung eines entsprechenden, geeigneten Promotors bzw. eines entsprechenden, geeigneten Expressionssystems).

Grundsätzlich ist es erfindungsgemäß bevorzugt, wenn die Inkubation in Schritt (ii) zumindest teilweise oder vollständig unter anaeroben Bedingungen erfolgt.

Je nach Mikroorganismus kann der Fachmann in Schritt (ii) für die Zwecke der vorliegenden Erfindung geeignete Umgebungsbedingungen schaffen und insbesondere ein geeignetes (Kultivierungs-)Medium bereitstellen. Die Kultivierung erfolgt vorzugsweise in LB- oder TB-Medium. Alternativ kann ein (komplexeres) Medium bestehend oder umfassend pflanzliche Rohstoffe, insbesondere aus Zitrus-, Grapefruit- und Orange-Pflanzen, verwendet werden. Die Kultivierung erfolgt beispielsweise bei einer Temperatur von mehr als 20 °C, bevorzugt von mehr als 25 °C, insbesondere von mehr als 30 °C (bevorzugt im Bereich von 30 bis 40 °C), was insbesondere die Bildung von Naringin Dihydrochalkon begünstigen bzw. die Ausbeute erhöhen kann. Weiterhin kann auch eine Temperatur zur Induktion (vgl. oben) von weniger als 40 °C, insbesondere von weniger als 35 °C (bevorzugt im Bereich von 20 bis 30 °C), die Bildung von Naringin Dihydrochalkon begünstigen bzw. die Ausbeute erhöhen.

Die Flavanonglykoside werden bezogen auf das (Kultivierungs-) Medium, das die transgenen Mikroorganismen enthält, in Schritt (ii) vorzugsweise in einer Menge von 0,1 mM bis 100 mM (mMol / L), bevorzugt von 0,5 bis 95 mM, besonders bevorzugt von 1 bis 90 mM, zu dem transgenen Mikroorganismus hinzugefügt. Hierbei können geeignete (Ko-)Solventien zum Einsatz kommen.

Falls zur Induktion (z.B. des lac-operons) ein oder mehrere geeignete Induktoren, z.B. IPTG oder Lactose , verwendet werden, ist es bevorzugt, den Induktor bezogen auf das (Kultivierungs-)Medium, das die transgenen Mikroorganismen enthält, in Schritt (ii) in einer Menge von 0,001 bis 1 mM, bevorzugt von 0,005 bis 0,9 mM, besonders bevorzugt von 0,01 bis 0,8 mM, einzusetzen, da hierbei besonders gute Ausbeuten erzielt werden können.

Zu Isolierung oder Aufreinigung der exprimierten Flavanonglykosid-Dihydrochalkone kann beispielsweise Extraktionen mit organischen Lösemitteln vorgenommen werden. Diese sind bevorzugt ausgewählt aus der folgenden Liste: Isobutan, 2-Propanol, Toluol, Methylacetat, Cyclohexan, 2-Butanol, Hexan, 1-Propanol, Light petroleum, 1,1,1,2-Tetrafluorethan, Methanol, Propan, 1-Butanol, Butan, Ethylmethylketon, Ethylacetat, Diethylether, Ethanol, Dibutylether, CO₂, tert. Butylmethylether, Aceton, Dichloromethan und N₂O. Besonders bevorzugt sind solche Lösungsmittel, die mit Wasser eine optisch erkennbare Phasengrenze ausbilden. Hiernach kann sich eine Entfernung des Restwassers im Lösemittel sowie die Entfernung des Lösemittels selbst anbieten, an die sich wiederum ein Rücklösen des Dihydrochalkons in einem (ggf. anderen) Lösemittel anschließen kann, welches z.B. für eine gegebenenfalls nachfolgende Kristallisation und Trocknung des Produkts geeignet ist. Alternativ oder ergänzend kann eine adsorptive, eine destillative und/oder eine chromatographische Aufreinigung erfolgen.

Alternativ können zur Isolierung oder Aufreinigung der gebildeten Flavanonglykosid-Dihydrochalkone Verfahren der Trocknung, insbesondere Verfahren der Vakuumbandtrockung, Sprühtrocknung, Destillation oder Lyophilisation der zellhaltigen oder-freien Fermentationslösung verwendet werden.

### TRANSGENE MIKROORGANISMEN

Unter einem "transgenen Mikroorganismus" ist im Zusammenhang mit der vorliegenden Erfindung ein gentechnisch veränderter bzw. modifizierter Mikroorganismus zu verstehen, in den gezielt mittels biotechnologischer Verfahren Nukleinsäure-Abschnitte (siehe Nukleinsäure-Abschnitte (A) und (B) wie hierin beschrieben) bzw. Gene eines anderen Organismus (sog. Transgene) eingeschleust worden sind.

Ein weiterer Gegenstand der Erfindung umfasst daher einen transgenen Mikroorganismus, enthaltend
(i) einen ersten Nukleinsäure-Abschnitt (A) enthaltend ein für eine bakterielle Chalkonisomerase kodierendes Gen, sowie
(ii) einen zweiten Nukleinsäure-Abschnitt (B), enthaltend ein für eine bakterielle Enoat-Reduktase kodierendes Gen,
wobei der Nukleinsäure-Abschnitt (A)
(1) eine Nukleinsäuresequenz gemäß SEQ ID NO:1 darstellt, bei der der Nukleinsäure-Abschnitt (A') gemäß SEQ ID NO:**3** herausgeschnitten worden ist, oder
(2) für eine Aminosäuresequenz gemäß SEQ ID NO:**2** kodiert, bei der der Aminosäuresäure-Abschnitt (A') gemäß SEQ ID NO:**4** herausgeschnitten worden ist.

Vorzugsweise handelt es sich dabei um einen fakultativ anaeroben Mikroorganismus, bevorzugt um ein Proteobakterium, insbesondere ein Enterobakterium, zum Beispiel der Gattung *Escherichia,* bevorzugt *E. coli,* insbesondere *E. coli* Rosetta, *E. coli* BL21 E.coli K12, E.coli MG1655,*E*. *coli* SE1 und deren Abkömmlinge, Hefen, zum Beispiel S. *cerevesiae* und *P. pastoris, K. lactis, H. polymorpha* sowie Pilze wie *Aspergillus spp.* oder *Trichoderma spp..*

Der erfindungsgemäße transgene Mikroorganismus zeichnet sich insbesondere dadurch aus, dass
(i) das für eine bakterielle Chalkonisomerase kodierende Gen für eine Chalkonisomerase aus einem Mikroorganismus aus dem Phylum der Firmicutes kodiert, und/oder
(ii) das für eine bakterielle Enoat-Reduktase kodierende Gen für eine Enoat-Reduktase aus einem Mikroorganismus aus dem Phylum der Firmicutes kodiert.

Methoden, um auf Basis der eingeschleusten Nukleinsäure-Abschnitte bzw. der Transgene eine Expression der gewünschten Aminosäuresequenzen bzw. der gewünschten Enzyme zu ermöglichen, sind dem Fachmann ebenfalls hinreichend bekannt, z.B. unter Verwendung eines regulatorischen Elements, insbesondere eines Promotors.

### VEKTOR

Ein weiterer Aspekt der vorliegenden Erfindung betrifft einen Vektor, d.h. ein Transportvesikel ("Genfähre"), insbesondere einen Plasmidvektor, zur Übertragung von Fremd-Nukleinsäure(n) in eine Empfängerzelle, insbesondere einen Plasmidvektor, der das Klonieren eines oder mehrerer Nukleinsäure-Abschnitte ermöglicht, enthaltend
(i) einen ersten Nukleinsäure-Abschnitt (A) enthaltend ein für eine bakterielle Chalkonisomerase kodierendes Gen, sowie
(ii) einen zweiten Nukleinsäure-Abschnitt (B), enthaltend ein für eine bakterielle Enoat-Reduktase kodierendes Gen,
wobei der Nukleinsäure-Abschnitt (A)
(1) eine Nukleinsäuresequenz gemäß SEQ ID NO:1 darstellt, bei der der Nukleinsäure-Abschnitt (A') gemäß SEQ ID NO:**3** herausgeschnitten worden ist, oder
(2) für eine Aminosäuresequenz gemäß SEQ ID NO:**2** kodiert, bei der der Aminosäuresäure-Abschnitt (A') gemäß SEQ ID NO:**4** herausgeschnitten worden ist.

### WIRTSZELLE

Die vorliegende Erfindung betrifft auch eine Wirtszelle, enthaltend ein oder mehrere identische oder unterschiedliche erfindungsgemäße Vektoren wie hierin beschrieben. Erfindungsgemäß bevorzugt ist eine Wirtszelle, die sowohl ein oder mehrere Vektoren mit einem Nukleinsäure-Abschnitt (A), enthaltend ein für eine bakterielle Chalkonisomerase kodierendes Gen, als auch ein oder mehrere Vektoren mit einem Nukleinsäure-Abschnitt (B), enthaltend ein für eine bakterielle Enoat-Reduktase kodierenden Gen.

Bei einer erfindungsgemäßen Wirtszelle handelt es sich vorzugsweise um einen erfindungsgemäß einzusetzenden bzw. einen erfindungsgemäßen Mikroorganismus (wie oben beschrieben). Die hierin beschriebenen erfindungsgemäßen Wirtszellen bzw. erfindungsgemäßen oder erfindungsgemäß einzusetzenden Mikroorganismen sind bzw. dienen vorzugsweise als (Produktions-)Stamm zur biotechnologischen Herstellung hierin beschriebener Dihydrochalkone, insbesondere von Naringin Dihydrochalkon.

### ZUBEREITUNGEN

Mit Hilfe des erfindungsgemäßen Verfahrens kann man so aus ein oder mehreren der oben genannten Flavanonglycoside, die in Flavanonglycosid-haltigen Lebensmitteln oder zur Lebensmittelherstellung geeigneten Zubereitungen enthalten sind, in die entsprechenden Dihydrochalkone umwandelt und damit als natürlich bezeichenbare Zubereitungen erhält, die dadurch charakterisiert sind, dass sie mindestens eines der oben genannten Dihydrochalkone enthält und damit weniger bitter und süßer schmecken als die Ausgangszubereitung.

In diesem Zusammenhang werden weiterhin Nahrungsmittelzubereitungen offenbart, die die nach dem erfindungsgemäßen Verfahren erhaltenen Flavanonglykosid-Dihydrochalkone enthalten.

Dabei werden die oben definierten Flavanonglycoside bevorzugt aus Pflanzenteilen, insbesondere Früchten oder Fruchtansätzen oder durch übliche Prozesse gewonnene Produkte z.B. getrocknete Früchte oder frische oder getrocknete Fruchtteile (z. B. Albedo, Flavedo), Direktsäfte, Saftfraktionen, Nebenströmen der Saftproduktion, etherischen Ölen, Oleoresinen, Saftkonzentraten, Fruchtpürees, Presskuchen, frischen oder getrockneten Blättern, der Gattungen *Citrus* oder *Poncirus* oder *Clymenia* oder *Eremocitrus* oder *Microcitrus* oder *Oxanthera* oder *Fortunella* oder *Eriodictyon* oder *Viscum* gefunden. Die oben definierten Flavanonglycoside können aus den durch übliche Prozesse gewonnene Produkten auch durch für die Lebensmittelproduktion erlaubten Lösungsmittel oder Lösungsmittelgemische oder durch pH-Änderung, bevorzugt alkalischer und dann saurer Behandlung, isoliert, extrahiert oder angereichert werden.

Die so gewonnen die Flavanonglycosid-haltigen Lebensmittel oder zur Lebensmittelherstellung geeigneten Zubereitungen werden dann mit den erfindungsgemäßen Verfahren in Dihydrochalkonglycosid-haltige, natürlich bezeichenbare Zubereitungen umgewandelt und können dann als solche verwendet werden oder nach Entfernung der Wirtszelle und/oder deren Bestandteilen und optionaler Aufkonzentration durch physikalische Prozesse als zur Lebensmittelherstellung geeigneten Zubereitung, bevorzugt als zur Lebensmittelherstellung geeigneten Zubereitung mit bittermaskierender oder süßverstärkender Wirkung eingesetzt werden.

Beispiele für geeignete Nahrungsmittel umfassen insbesondere Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen sowie Instantgetränke (beispielsweise Instant-Kakao-Getränke, Instant-Tee-Getränke, Instant-Kaffeegetränke, Instant-Fruchtgetränke).

Besonders bevorzugt sind dabei als Flavanonglycosid-haltigen Lebensmittel Zitrussäfte, insbesondere Orangensäfte, die einen hohen Gehalt an Naringin enthalten und durch das erfindungsgemäße Verfahren in erfindungsgemäße Naringindihydrochalkon-haltige Orangensäfte umgewandelt werden können.

### BEISPIELE

### BEISPIEL 1

### Klonierungsstrategie

Das Herausschneiden der Sequenz des Lids aus dem Gen der CHI wurde mittels der *FastCloning-Methode* durchgeführt. Hierfür erfolgte eine PCR mit den unten genannten Primer auf pET28_CHI (Denaturierung: 30s 95°C, Annealing: 30s 50°C, Elongation: 6.5 min 72°C). Anschließend wurde das Ausgangs-Plasmid mit *Dpn*l verdaut.
Forward Primer: 5'-GATCCCGGCAGCAGCAGAAGGAAATCC-3'
Reverse Primer: 5'-GGATTTCCTTCTGCTGCTGCCGGGATC-3'

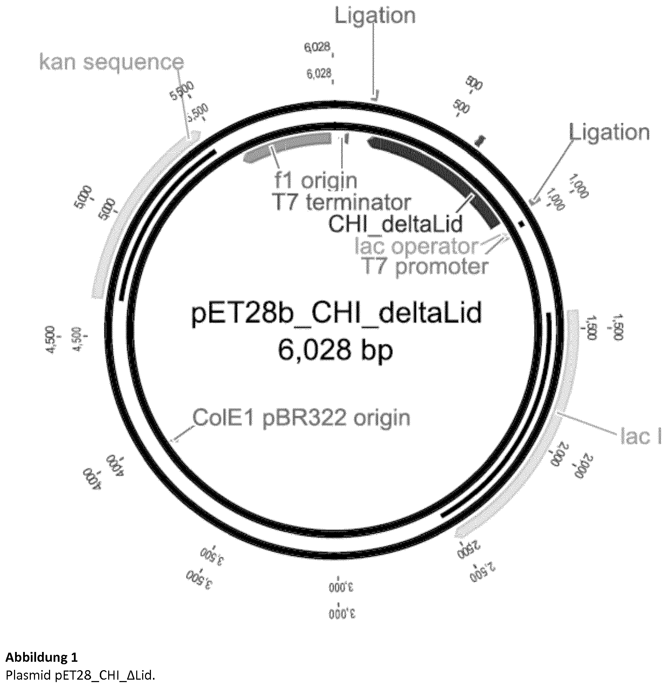

Nach erfolgreicher Klonierung wurde das Plasmid pET28_CHI_ΔLid **(****Abbildung 1****)** zusammen mit dem Plasmid pET22_ERED in *E. coli* BL21 transformiert.

### BEISPIEL 2

### Expression der Doppeltransformation

200 mL LB_{kan,amp} wurden mit 1% (v/v) einer Übernachtkultur angeimpft und bei 37°C bis auf eine OD≥1 angezogen. Dann erfolgte die Induktion der Expression mit 0.1 mM IPTG. Die Expression des Proteins erfolgte 21h bei 20°C.

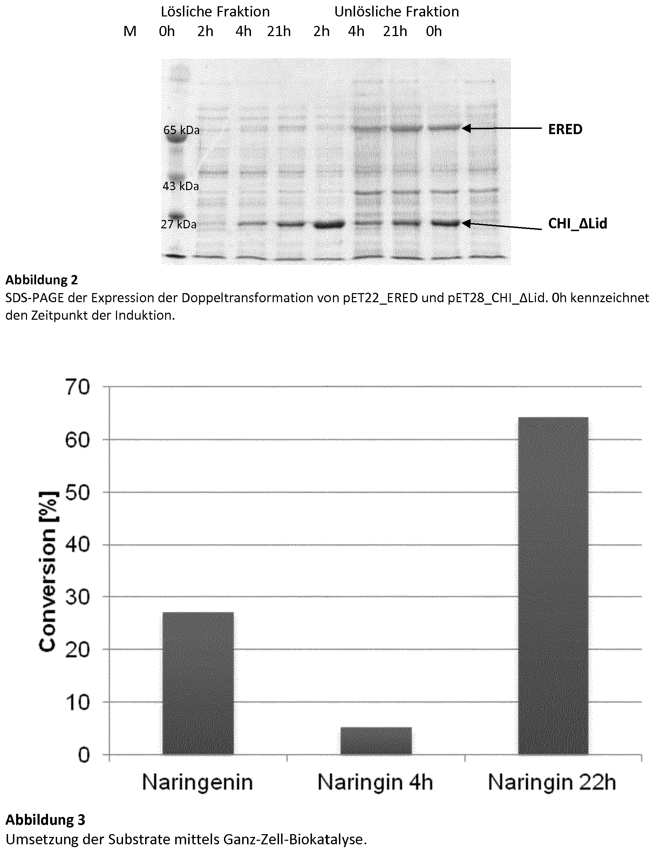

Wie in **Abbildung 2** zu sehen ist, erfolgte die Expression der CHI_ΔLid sowohl in der löslichen als auch in der unlöslichen Fraktion, während die ERED nur in der unlöslichen Form exprimierbar ist.

### BEISPIEL 3

### Ganz-Zell-Biokatalysen

Nach der Ernte der Zellen wurden diese mit 20 mM TRIS-HCl pH 7.5 auf eine OD=150 normiert. Zur Durchführung der Biokatalysen wurden jeweils 490 µL der Zellsuspension mit 10 µL Naringin-Lösung (Stammlösung: 7.5 mM Naringin gelöst in 1,2-Propylenglykol; Endkonzentration: 150 µM) in ein 2-mL-Eppendorf-Reaktionsgefäß gegeben und mit Stickstoff begast. Die Biokatalysen wurden bei 23°C, 900 rpm und 22h durchgeführt. Die Extraktion der Substrate erfolgte zweimal mit Ethylacetat.

Nach 22h konnte eine 65%ige Umsetzung des Naringins beobachtet werden **(Abbildung 3).**

### BEISPIEL 4

### Entbitterung von Naringin-haltigem Orangensaft

Ein Orangensaft mit einer Konzentration von 100 ppm Naringin wird mit den in Beispiel 3 beschriebenen Zellen inkubiert und bei 23°C unter Rühren 48 inkubiert. Der Gehalt des Naringins wurde auf 50 ppm gesenkt und gleichzeitig bildet sich Naringindihydrochalkon.

## Patentansprüche

1. Verfahren zur Herstellung von Flavanonglykosid-Dihydrochalkonen, umfassend die Schritte:
(a) Bereitstellen eines transgenen Mikroorganismus, enthaltend
(i) einen ersten Nukleinsäure-Abschnitt (A) enthaltend ein für eine bakterielle Chalkonisomerase kodierendes Gen, sowie
(ii) einen zweiten Nukleinsäure-Abschnitt (B), enthaltend ein für eine bakterielle Enoat-Reduktase kodierendes Gen,
(b) Hinzufügen eines oder mehrere Flavanonglykoside zu dem transgenen Mikroorganismus,
(c) Kultivierung des transgenen Mikroorganismus unter Bedingungen, die die gleichzeitig Isomerisierung und Reduktion des Flavanonglykosids zum Flavanonglykosid-Dihydrochalkon ermöglichen, sowie gegebenenfalls
(d) Isolierung und Aufreinigung des Endproduktes.
wobei der Nukleinsäure-Abschnitt (A)
(1) eine Nukleinsäuresequenz gemäß SEQ ID NO:1 darstellt, bei der der Nukleinsäure-Abschnitt (A') gemäß SEQ ID NO:**3** herausgeschnitten worden ist, oder
(2) für eine Aminosäuresequenz gemäß SEQ ID NO:**2** kodiert, bei der der Aminosäuresäure-Abschnitt (A') gemäß SEQ ID NO:**4** herausgeschnitten worden ist.

2. Verfahren nach Anspruch 1, **dadurch gekennzeichnet, dass** der transgene Mikroorganismus ein fakultativ anaerober Mikroorganismus ist.

3. Verfahren nach den Ansprüchen 1 und/oder 2, **dadurch gekennzeichnet, dass** das für eine bakterielle Chalkonisomerase kodierende Gen für eine Chalkonisomerase aus einem Mikroorganismus aus dem Phylum der Firmicutes kodiert.

4. Verfahren nach mindestens einem der Ansprüche 1 bis 3, **dadurch gekennzeichnet, dass** das für eine bakterielle Enoat-Reduktase kodierende Gen für eine Enoat-Reduktase aus einem Mikroorganismus aus dem Phylum der Firmicutes kodiert.

5. Verfahren nach mindestens einem der Ansprüche 1 bis 4, **dadurch gekennzeichnet, dass** der Nukleinsäure-Abschnitt (B) eine Nukleotidsequenz gemäß SEQ ID NO**:5** oder SEQ ID NO:**7** darstellt.

6. Verfahren nach mindestens einem der Ansprüche 1 bis 5, **dadurch gekennzeichnet, dass** die bakterielle Chalkonisomerase eine Nukleotidsequenz gemäß SEQ ID NO:**1** enthält, bei der der Nukleinsäure-Abschnitt (A') gemäß SEQ ID NO**:3** herausgeschnitten worden ist.

7. Verfahren nach mindestens einem der Ansprüche 1 bis 6, **dadurch gekennzeichnet, dass** die bakterielle Enoat-Reduktase eine Nukleotidsequenz gemäß SEQ ID NO:**5** oder SEQ ID NO**:7** enthält.

8. Verfahren nach mindestens einem der Ansprüche 1 bis 7, **dadurch gekennzeichnet, dass** der in den transgenen Mikroorganismus exprimierte Aminosäure-Abschnitt (B) eine Aminosäuresequenz gemäß SEQ ID NO:**6** darstellt.

9. Verfahren nach mindestens einem der Ansprüche 1 bis 8, **dadurch gekennzeichnet, dass** die bakterielle Enoat-Reduktase eine Aminosäuresequenz gemäß SEQ ID NO:**6** enthält.

10. Verfahren nach mindestens einem der Ansprüche 1 bis 9, **dadurch gekennzeichnet, dass** die Flavanonglykoside ausgewählt sind aus der Gruppe, die gebildet wird Naringin, Narirutin, Prunin (Naringin-7-O-glucosid), Hesperidin, Neohesperidin, Hesperetin-7-O-glucosid, Eriodictyolglycoside wie Eriocitrin, Neoeriocitrin, Eriodictyol-7-O-glucosid, Homoeriodictyolglycoside wie Homoeriodictyol-7-O-glucosid, Sterubinglycoside, Sakuranetinglycoside, Isosakuranetinglycoside, 4',7-Dihydroxy-flavanonglykoside, 4',7-Dihydroxy-3'-methoxy-flavanonglycoside, 3',7-Dihydroxy-4'-methoxy-flavanonglycoside, 3',4',7-Trihydroxy-flavanonglycoside sowie deren Gemischen.

11. Transgener Mikroorganismus, enthaltend
(i) einen ersten Nukleinsäure-Abschnitt (A) enthaltend ein für eine bakterielle Chalkonisomerase kodierendes Gen, sowie
(ii) einen zweiten Nukleinsäure-Abschnitt (B), enthaltend ein für eine bakterielle Enoat-Reduktase kodierendes Gen.
wobei der Nukleinsäure-Abschnitt (A)
(1) eine Nukleinsäuresequenz gemäß SEQ ID NO:1 darstellt, bei der der Nukleinsäure-Abschnitt (A') gemäß SEQ ID NO**:3** herausgeschnitten worden ist, oder
(2) für eine Aminosäuresequenz gemäß SEQ ID NO**:2** kodiert, bei der der Aminosäuresäure-Abschnitt (A') gemäß SEQ ID NO**:4** herausgeschnitten worden ist.

12. Mikroorganismus nach Anspruch 11, **dadurch gekennzeichnet, dass** es sich um einen fakultativ anaeroben Mikroorganismus handelt.

13. Mikroorganismus nach den Ansprüchen 11 und/oder 12, **dadurch gekennzeichnet, dass**
(i) das für eine bakterielle Chalkonisomerase kodierende Gen für eine Chalkonisomerase aus einem Mikroorganismus aus dem Phylum der Firmicutes kodiert, und/oder
(ii) das für eine bakterielle Enoat-Reduktase kodierende Gen für eine Enoat-Reduktase aus einem Mikroorganismus aus dem Phylum der Firmicutes kodiert.

14. Vektor, enthaltend
(i) einen ersten Nukleinsäure-Abschnitt (A) enthaltend ein für eine bakterielle Chalkonisomerase kodierendes Gen, sowie
(ii) einen zweiten Nukleinsäure-Abschnitt (B), enthaltend ein für eine bakterielle Enoat-Reduktase kodierendes Gen.
wobei der Nukleinsäure-Abschnitt (A)
(1) eine Nukleinsäuresequenz gemäß SEQ ID NO:1 darstellt, bei der der Nukleinsäure-Abschnitt (A') gemäß SEQ ID NO:**3** herausgeschnitten worden ist, oder
(2) für eine Aminosäuresequenz gemäß SEQ ID NO**:2** kodiert, bei der der Aminosäuresäure-Abschnitt (A') gemäß SEQ ID NO**:4** herausgeschnitten worden ist.

15. Wirtszelle, enthaltend mindestens einen Vektor nach Anspruch 14.

## Claims

1. A process for the production of flavanone glycoside dihydrochalcones encompassing the following steps:
(a) provision of a transgenic micro-organism comprising
(i) a first nucleic acid section (A) containing a gene coding for a bacterial chalconeisomerase; and
(ii) a second nucleic acid section (B) containing a gene coding for a bacterial enoate-reductase;
(b) addition of one or more flavanone glycosides to said transgenic micro-organism;
(c) cultivation of said transgenic micro-organism under conditions allowing simultaneously isomerisation and reduction of said flavanone glycosides to said flavanone glycoside dihydrochalcones; and optionally
(d) isolation and purification of the final product,
whereby said nucleic acid sequence (A)
(1) represents a nucleic acid sequence according to SEQ ID NO:1, wherein a nucleic acid section (A') according to SEQ ID NO:3 has been cut out; or
(2) is coding for an amino acid sequence according to SEQ ID NO:2, wherein a nucleic acid sequence (A') according to SEQ ID NO:4 has been cut out.

2. The process of claim 1, **characterized in that** said transgenic micro-organism represents a facultative anaerobic micro-organism.

3. The process of claims 1 and/or 2, **characterized in that** said gene coding for a bacterial chalconeisomerase is coding for a chalconeisomerase obtained from a micro-organism from the phylum of firmicutes.

4. The process of any of the claims 1 to 3, **characterized in that** said gene coding for a bacterial enoate-reductase is coding for an enoate-reductase obtained from a micro-organism from the phylum of firmicutes.

5. The process of any of the claims 1 to 4, **characterized in that** said nucleic acid section (B) represents a nucleotide sequence according to SEQ ID NO:5 or SED ID NO:7.

6. The process of any of the claims 1 to 5, **characterized in that** said bacterial chalconeisomerase contains a nucleotide sequence according to SEQ ID 1, wherein a nucleic acid section (A') according to SEQ ID NO:3 has been cut out.

7. The process of any of the claims 1 to 6, **characterized in that** said bacterial enoate-reductase contains a nucleotide sequence according to SEQ ID NO:5 or SEQ IS NO:7.

8. The process of any of the claims 1 to 7, **characterized in that** said amino acid section (B) which is expressed into the transgenic micro-organism represents an amino acid sequence according to SEQ ID NO:6.

9. The process of any of the claims 1 to 8, **characterized in that** said bacterial enoate-reductase contains an amino acid sequence according to SEQ ID NO:6.

10. The process of any of the claims 1 to 9, **characterized in that** said flavone glycosides are selected from the group consisting of naringine, narirutine, prunine (Naringine-7-O-glucoside), hesperidine, neohesperidine, hesperetine-7-O-glucoside, eriodictyol glycoside as for example eriocitrine, neoeriocitrine, eriodictyol-7-O-glucoside, homoeriodictyol glycoside as for example homoeriodictyol-7-O-glucoside, sterubine glycoside, sakuranetine glycoside, isosakuranetine glycoside, 4',7-Dihydroxy-flavanone glycoside, 4',7-Dihydroxy-3'-methoxy-flavanone glycoside, 3',7-Dihydroxy-4'-methoxy-flavanone - glycoside, 3',4',7-Trihydroxy-flavanone glycoside and their mixtures.

11. Transgenic micro-organism comprising
(i) a first nucleic acid section (A) containing a gene coding for a bacterial chalconeisomerase; and
(ii) a second nucleic acid section (B) containing a gene coding for a bacterial enoate reductase;
whereby said nucleic acid sequence (A)
(1) represents a nucleic acid sequence according to SEQ ID NO:1, wherein a nucleic acid section (A') according to SEQ ID NO:3 has been cut out; or
(2) is coding for an amino acid sequence according to SEQ ID NO:2, wherein a nucleic acid sequence (A') according to SEQ ID NO:4 has been cut out.

12. A micro-organism according to claim 11, **characterized in that** it represents a facultative anaerobic micro-organism.

13. The micro-organism according to claim 11 and/or 12, **characterized in that**
(i) said gene coding for a bacterial chalconeisomerase is coding for a chalconeisomerase obtained from a micro-organism from the phylum of firmicutes; and/or
(ii) said gene coding for a bacterial enoate-reductase is coding for an enoate-reductase obtained from a micro-organism from the phylum of firmicutes.

14. A vector containing
(i) a first nucleic acid section (A) containing a gene coding for a bacterial chalconeisomerase; and
(ii) a second nucleic acid section (B) containing a gene coding for a bacterial enoate reductase;
whereby said nucleic acid sequence (A)
(1) represents a nucleic acid sequence according to SEQ ID NO:1, wherein a nucleic acid section (A') according to SEQ ID NO:3 has been cut out; or
(2) is coding for an amino acid sequence according to SEQ ID NO:2, wherein a nucleic acid sequence (A') according to SEQ ID NO:4 has been cut out.

15. A host cell comprising at least one vector according to claim 14.

## Revendications

1. Procédé pour la préparation de dihydrochalcones de glycosides de flavanone, comprenant les étapes de :
(a) préparation d'un micro-organisme transgénique, contenant
(i) une première section (A) d'acide nucléique contenant un gène codant pour une chalcone-isomérase bactérienne ainsi que
(ii) une deuxième section (B) d'acide nucléique contenant un gène codant pour une énoate-réductase bactérienne,
(b) addition d'un ou de plusieurs glycosides de flavanone au micro-organisme transgénique,
(c) culture du micro-organisme transgénique dans des conditions qui permettent en même temps l'isomérisation et la réduction du glycoside de flavanone en dihydro-chalcone de glycoside de flavanone, ainsi que le cas échéant
(d) isolement et purification du produit final,
la section (A) d'acide nucléique
(1) représentant une séquence d'acide nucléique selon la séquence SEQ ID NO: 1, dont la section (A') d'acide nucléique selon la séquence SEQ ID NO : 3 a été excisée, ou
(2) codant pour une séquence d'acides aminés selon la séquence SEQ ID NO : 2, dont la section (A') d'acides aminés selon la séquence SEQ ID NO : 4 a été excisée.

2. Procédé selon la revendication 1, **caractérisé en ce que** le micro-organisme transgénique est un micro-organisme facultativement anaérobie.

3. Procédé selon les revendications 1 et/ou 2, **caractérisé en ce que** le gène codant pour une chalcone-isomérase bactérienne code pour une chalcone-isomérase provenant d'un micro-organisme du phylum des Firmicutes.

4. Procédé selon au moins l'une quelconque des revendications 1 à 3, **caractérisé en ce que** le gène codant pour une énoate-réductase bactérienne code pour une énoate-réductase provenant d'un micro-organisme du phylum des Firmicutes.

5. Procédé selon au moins l'une quelconque des revendications 1 à 4, **caractérisé en ce que** la section (B) d'acide nucléique représente une séquence d'acide nucléique selon la séquence SEQ ID NO:5 ou la séquence SEQ ID NO:7.

6. Procédé selon au moins l'une quelconque des revendications 1 à 5, **caractérisé en ce que** la chalcone-isomérase bactérienne contient une séquence d'acide nucléique selon la séquence SEQ ID NO : 1, dont la section (A') d'acide nucléique selon la séquence SEQ ID NO : 3 a été excisée.

7. Procédé selon au moins l'une quelconque des revendications 1 à 6, **caractérisé en ce que** l'énoate-réductase bactérienne contient une séquence d'acide nucléique selon la séquence SEQ ID NO:5 ou la séquence SEQ ID NO:7.

8. Procédé selon au moins l'une quelconque des revendications 1 à 7, **caractérisé en ce que** la section (B) d'acides aminés exprimée dans le micro-organisme transgénique représente une séquence d'acides aminés selon la séquence SEQ ID NO : 6.

9. Procédé selon au moins l'une quelconque des revendications 1 à 8, **caractérisé en ce que** l'énoate-réductase bactérienne contient une séquence d'acides aminés selon la séquence SEQ ID NO:6.

10. Procédé selon au moins l'une quelconque des revendications 1 à 9, **caractérisé en ce que** les glycosides de flavanone sont choisis dans le groupe formé par la naringine, la narirutine, la prunine (naringine-7-O-glucoside), l'hespéridine, la néohespéridine, l'hespérétine-7-O-glucoside, les glycosides d'ériodictyol, tels que l'ériocitrine, la néo-ériocitrine, l'ériodictyol-7-O-glucoside, les glycosides d'homo-ériodictyol, tels que l'homoériodictyol-7-O-glucoside, les glycosides de stérubine, les glycosides de sakuranétine, les glycosides d'isosakuranétine, les glycosides de 4',7-dihydroxyflavanone, les glycosides de 4',7-dihydroxy-3'-méthoxyflavanone, les glycosides de 3',7-dihydroxy-4'-méthoxyflavanone, les glycosides de 3',4',7-trihydroxyflavanone ainsi que leurs mélanges.

11. Micro-organisme transgénique, contenant
(i) une première section (A) d'acide nucléique contenant un gène codant pour une chalcone-isomérase bactérienne ainsi que
(ii) une deuxième section (B) d'acide nucléique contenant un gène codant pour une énoate-réductase bactérienne,
la section (A) d'acide nucléique
(1) représentant une séquence d'acide nucléique selon la séquence SEQ ID NO: 1, dont la section (A') d'acide nucléique selon la séquence SEQ ID NO : 3 a été excisée, ou
(2) codant pour une séquence d'acides aminés selon la séquence SEQ ID NO : 2, dont la section (A') d'acides aminés selon la séquence SEQ ID NO : 4 a été excisée.

12. Micro-organisme selon la revendication 11, **caractérisé en ce qu'**il s'agit d'un micro-organisme facultativement anaérobie.

13. Micro-organisme selon les revendications 11 et/ou 12, **caractérisé en ce que**
(i) le gène codant pour une chalcone-isomérase bactérienne code pour une chalcone-isomérase provenant d'un micro-organisme du phylum des Firmicutes et/ou
(ii) le gène codant pour une énoate-réductase bactérienne code pour une énoate-réductase provenant d'un micro-organisme du phylum des Firmicutes.

14. Vecteur, contenant
(i) une première section (A) d'acide nucléique contenant un gène codant pour une chalcone-isomérase bactérienne ainsi que
(ii) une deuxième section (B) d'acide nucléique contenant un gène codant pour une énoate-réductase bactérienne,
la section (A) d'acide nucléique
(1) représentant une séquence d'acide nucléique selon la séquence SEQ ID NO : 1, dont la section (A') d'acide nucléique selon la séquence SEQ ID NO : 3 a été excisée, ou
(2) codant pour une séquence d'acides aminés selon la séquence SEQ ID NO : 2, dont la section (A') d'acides aminés selon la séquence SEQ ID NO : 4 a été excisée.

15. Cellule hôte contenant au moins un vecteur selon la revendication 14.


### SEQUENCE LISTING

<110> Symrise AG
<120> Verfahren zur biotechnologischen Herstellung von Flavanonglykosid-Dihydrochalkonen
<130> C 3275 EP
<140> EPEp14175312.9 <141> 2014-07-01
<150> EP14175312.9 <151> 2014-07-01
<160> 7
<170> BiSSAP 1.3
<210> 1
   <211> 852
   <212> DNA
   <213> Eubacterium ramulus
<400> 1
   atggcagatt tcaaattcga accaatgaga agtcttatct acgttgactg cgtaagcgaa      60
   gactacagac caaaacttca gagatggatt tataaagtac atattccgga cagcatctct     120
   cagtttgagc cgtatgttac caaatatgca ttttatccgt ccttcccgat tccaccacag     180
   ggtgatcgtt tcggatacgc aagaatgcag ctgacagagc atcactggtt agtaagcgac     240
   cttgatcctc gtcttgagat caaagcaatc gctgagacat tcccgatgga cgtacttgta     300
   tggcagggac agatcccggc agcagctcat acagacgctc agatcgattc tgacggagat     360
   gcaggaaatg cagcccgtaa atccaacaat gcagaaggaa atccatttat ctttgcattc     420
   cttccgatgt ggtgggagaa agacctgaaa ggaaaaggac gtacgatcga ggacggcgca     480
   aactatcgtt tcaatatgac tatcggtttc ccagaaggcg tagacaaagc agagggagag     540
   aaatggttat ttgagaaagt agttccgatt cttcaggcag ctccggagtg tacacgtgta     600
   cttgcaagtg ccgtaaagaa agacatcaac ggatgcgtaa tggattgggt acttgaaatc     660
   tggtttgaga atcagtccgg atggtacaaa gtaatggtag atgacatgaa agcacttgaa     720
   aaaccgtcat gggctcagca ggatgctttc ccgttcctga aaccatacca caatgtttgc     780
   agtgcagcag ttgctgatta tacaccaagc aacaaccttg caaattatcg tggatacatc     840
   accatgagat aa                                                         852
<210> 2
   <211> 187
   <212> DNA
   <213> Eubacterium ramulus
<400> 2
   madkmrsyvd cvsdyrkrwy kvhdssyvtk yaysgdrgya rmthhwvsdd rkaatmdvvw 60
   gaaahtdads dgdagnaark snnagnamww kdkgkgrtdg anyrnmtggv dkagkwkvva 120
   actrvasavk kdngcvmdwv wnsgwykvmv ddmkakswad akyhnvcsaa vadytsnnan 180
   yrgytmr 187
<210> 3
   <211> 66
   <212> DNA
   <213> Eubacterium ramulus
<400> 3
   catacagacg ctcagatcga ttctgacgga gatgcaggaa atgcagcccg taaatccaac 60
   aatgca 66
<210> 4
   <211> 20
   <212> DNA
   <213> Eubacterium ramulus
<400> 4
   htdadsdgda gnaarksnna 20
<210> 5
   <211> 2037
   <212> DNA
   <213> Eubacterium ramulus
<400> 5
   atggcagaaa aaaatcagta ttttccacat ttgtttgagc cgttaaaagt tggttcaaag 60
   acaattaaga accgtattga ggcagcaccg gctttatttg cattcgagca ttatatcgaa     120
   ctgaatccgg atccgtttgg ctataccaca ccggttccgg agcgtgcgtt ccgtatgctg     180
   gaggcaaagg caaaaggagg ggcaggaatt gtatgtctgg gtgagttaag cccgaatcat     240
   gagtatgaca aacggtttcc gtttgaaccg tatcttgatt ttacatccag atcagataag     300
   cagtttgaaa ttatgaagga aactgcggag atgatcaaaa gctatggggc atttccgatg     360
   ggcgagctgc tttcctgcgg tgaaatcaag acaaatatcg gagatggtat caatccgaag     420
   ggaccatctg agaaagatct tccggatggc tctcatgtgg aggcgtttac aaaagaagag     480
   attttaagct gctatcagga ttatgtaact gcatgtaaat ggtttcaggc agcaggctgg     540
   gaaggcatta tgatccactg cggacatggc tggcttccgg cacagttcct gtctccgcaa     600
   tacaataaac gtaccgatga gtatggtgga tcttttgaaa acagagcaag atttactgtt     660
   gatctgttaa aaactgttcg tgaagctatg ggaccggact tcgtgatcga gatccgtgtc     720
   agcagctctg agcatttacc gggcggatta gagctggaag atgctgtaaa ttattgtaaa     780
   ctgtgtgagc catacattga tatgatccat gtctcctgtg gtcattacct gagttcttcc     840
   agaagttggg agttcacaac tgcttatgca ccgcatggtc cgaatattga accggcagct     900
   gttatcaaac agaacgtatc cattccggtt gcggcagtcg gcggcatcaa ttctccggaa     960
   caggcggaag aggcaatcgc gtcaggaaaa atcgatatgg tatccatggg acgtcagttc    1020
   tttgcagatc cggcatttcc aaacaaggca aaagaagggc atgcagatga gatccgtcgc    1080
   tgtctgcgct gcggaagatg ctatccgggt ccgtccggcg agcatgaaac agagatctgg    1140
   acggtgaaat tcccaccact ggattcctgt accatcaatc catatgatgt atggccggca    1200
   tctcatcata aagtccttcc ggaccgcatg ccgaaaccgg aagcaagccg taaggtattg    1260
   gtagtaggcg gcggctgtgg cggtctgcag acagcgatca cagcatcaga cagaggtcat    1320
   caggtaatcc tgtgcgaaaa atccggagta ttaggcggtc tgatcaattt tacggatcat    1380
   acggatcata aagtagatat cagaaacttc aaagatctgc tgatccgcga tgtggagaaa    1440
   cgtccgatcg aagtaagatt aaactgtgaa gtaacaccgg aactcatcag agaaattgct    1500
   ccggaagcag ttgtactggc cgtcggatcc gatgatctga tccttccaat cgagggaatt    1560
   gaaaatgcgg taacagcaat ggacgtatac agcaatgact ttgcaggtct tggaaagagc 1620
   accatcgtac tcggtggcgg tctggttggc tgtgaggcag ccgcagatta tattgatcac 1680
   ggtgtagaga caacgattgt tgaaatgaaa ggtgcgctga tgccggagac aaccggtctg 1740
   taccgtacag ctgtacatga tttcatcgac aaaaacggcg gcaaatacga agtaaatgca 1800
   aaagttgtca aagttggcaa agattttgtg gtagcggaac aagatgggaa agagattacc 1860
   atcaaagcag attctgttgt caatgcaatg ggacgccgtg cgcatgcgac agaagcactt 1920
   gagacagcta tcaaagaagc tggtattccg gtatggaaga tcggtgactg tgtccgtgcg 1980
   cgtcagatcg gtgatgcggt aagagaaggc tggaccgcag caatggaaat tatctaa 2037
<210> 6
   <211> 447
   <212> DNA
   <213> Eubacterium ramulus
<400> 6
   maknyhkvgs ktknraaaah yndgyttvra rmakakggag vcgsnhydkr ydtsrsdkmk 60
   tamksygamg scgktngdgn kgskddgshv atkscydyvt ackwaagwgm hcghgwasyn 120
   krtdyggsnr artvdktvra mgdvrvsssh ggdavnyckc ydmhvscghy sssrswttay 180
   ahgnaavknv svaavggnsa aasgkdmvsm gradankakg hadrrcrcgr cygsghtwtv 240
   kdsctnydvw ashhkvdrmk asrkvvvggg cggtatasdr ghvcksgvgg ntdhtdhkvd 300
   rnkdrdvkrv rncvtraavv avgsddgnav tamdvysnda ggkstvgggv gcaaadydhg 360
   vttvmkgamt tgyrtavhdd knggkyvnak vvkvgkdvva dgktkadsvv namgrrahat 420
   atakagvwkg dcvrargdav rgwtaam 447
<210> 7
   <211> 2037
   <212> DNA
   <213> Eubacterium ramulus
<400> 7
   atggcagaaa agaaccaata cttcccgcac ctgtttgaac cgctgaaagt cggctctaaa 60
   accattaaaa atcgcatcga agcagcaccg gccctgtttg cattcgaaca ttatatcgaa 120
   ctgaacccgg acccgtttgg ttacaccacg ccggtgccgg aacgtgcatt ccgtatgctg     180
   gaagccaaag caaaaggcgg tgccggcatt gtttgtctgg gtgaactgag cccgaatcac     240
   gaatatgata aacgctttcc gttcgaaccg tacctggatt ttaccagccg ttctgacaaa     300
   cagttcgaaa ttatgaaaga aacggcagaa atgatcaaaa gctatggcgc ttttccgatg     360
   ggtgaactgc tgtcgtgcgg tgaaatcaaa accaacattg gcgatggtat caatccgaaa     420
   ggcccgtcag aaaaagatct gccggacggt tcgcatgtgg aagccttcac caaagaagaa     480
   atcctgtcat gttaccagga ttacgttacg gcatgcaaat ggttccaagc ggccggctgg     540
   gaaggtatta tgatccattg tggccacggt tggctgccgg cgcagtttct gagcccgcaa     600
   tataacaaac gcaccgatga atacggcggt tcttttgaaa atcgtgcgcg cttcaccgtc     660
   gatctgctga aaacggtgcg tgaagcgatg ggcccggact tcgtgattga aatccgtgtt     720
   agctctagtg aacatctgcc gggcggtctg gaactggaag atgcggtgaa ctattgcaaa     780
   ctgtgtgaac cgtacattga catgatccat gttagttgcg gccactatct gtcctcatcg     840
   cgctcctggg aatttaccac ggcttacgcg ccgcacggtc cgaacatcga accggcagct     900
   gtcattaaac agaatgtgag catcccggtt gcagcagtcg gcggtatcaa ctctccggaa     960
   caagcggaag aagccattgc aagtggcaaa atcgatatgg ttagcatggg ccgtcagttt    1020
   ttcgctgacc cggcgtttcc gaataaagca aaagaaggcc atgctgatga aattcgtcgc    1080
   tgcctgcgtt gtggtcgctg ctatccgggc ccgagtggtg aacacgaaac cgaaatctgg    1140
   acggtgaaat tcccgccgct ggatagttgt accattaacc cgtacgacgt gtggccggca    1200
   tcccatcaca aagttctgcc ggatcgcatg ccgaaaccgg aagcgtcccg taaagttctg    1260
   gtggttggcg gtggctgtgg tggtctgcag accgcaatca cggcatcaga ccgcggccat    1320
   caagtcattc tgtgcgaaaa atcgggtgtg ctgggtggcc tgattaactt taccgatcat    1380
   acggaccaca aagttgatat tcgcaatttc aaagatctgc tgatccgtga cgtcgaaaaa    1440
   cgcccgattg aagttcgtct gaattgtgaa gtcaccccgg aactgattcg tgaaatcgct    1500
   ccggaagcag tcgtgctggc agtgggcagt gatgacctga ttctgccgat cgaaggtatt    1560
   gaaaacgccg ttaccgcaat ggatgtctat agcaatgact ttgccggcct gggtaaatct    1620
   acgatcgtgc tgggtggcgg tctggttggc tgcgaagcag ctgcggatta tatcgatcat    1680
   ggcgtggaaa ccacgattgt tgaaatgaaa ggcgcactga tgccggaaac cacgggtctg    1740
   tatcgtaccg ctgtgcacga ttttattgac aaaaacggcg gtaaatacga agtcaatgcc    1800
   aaagttgtca aagtgggcaa agatttcgtg gttgcagaac aggacggtaa agaaattacc    1860
   atcaaagcgg attctgtcgt gaatgcgatg ggccgtcgcg ctcacgcaac cgaagctctg    1920
   gaaacggcga ttaaagaagc cggcatcccg gtttggaaaa ttggtgattg cgtccgtgcc    1980
   cgccaaatcg gtgacgcagt tcgtgaaggc tggacggctg caatggaaat catctaa       2037